# EUROPEAN PATENT SPECIFICATION

(11) **EP 2 802 310 B1**
(45) Date of publication and mention of the grant of the patent: **21.11.2018**
(21) Application number: 13705571.1
(22) Date of filing: 10.01.2013
(51) Int. Cl.: A61K 8/9794, A61Q 19/06, A61K 36/9068, A61K 8/35, A61K 8/49, A61Q 19/08

(54) **NEW USE OF ZERUMBONE AND COMPOSITIONS COMPRISING ZERUMBONE**
NEUE VERWENDUNG VON ZERUMBON UND ZUSAMMENSETZUNGEN MIT ZERUMBON
NOUVELLE UTILISATION DU ZÉRUMBONE, ET COMPOSITIONS COMPRENANT LE ZÉRUMBONE

(30) Priority: 10.01.2012 FR 1250241; 09.07.2012 FR 1256579
(43) Date of publication of application: 19.11.2014
(73) Proprietor: Sederma, 78610 Le Perray en Yvelines (FR)
(72) Inventor: FOURNIAL, Arnaud, 75016 Paris (FR); GRIZAUD, Claire-Marie, Houston, TX, 77088 (US); MONDON, Philippe, 92120 Montrouge (FR)
(86) International application number: PCT/IB2013/050226
(87) International publication number: WO 2013/105048

(56) References cited:
- WO-A2-2006/034463
- CN-A- 1 276 176
- CHIA JU CHANG ET AL: "Regulation of lipid disorders by ethanol extracts from high-fat diet-induced rats", FOOD CHEMISTRY, ELSEVIER LTD, NL, vol. 132, no. 1, 3 November 2011 (2011-11-03), pages 460-467, XP028344747, ISSN: 0308-8146, DOI: 10.1016/J.FOODCHEM.2011.11.022 [retrieved on 2011-11-11]
- JOHN H. BEATTIE ET AL: "Ginger phytochemicals mitigate the obesogenic effects of a high-fat diet in mice: A proteomic and biomarker network analysis", MOLECULAR NUTRITION & FOOD RESEARCH, vol. 55, no. S2, 30 August 2011 (2011-08-30), pages S203-S213, XP055039806, ISSN: 1613-4125, DOI: 10.1002/mnfr.201100193
- Innovadex: "Resistem by Sederma", , 2011, XP002684959, Retrieved from the Internet: URL:http://www.innovadex.com/documents/112 3145.pdf?bs=1241&b=193188&st=1&sl=19965761 &crit=a2V5d29yZDpbcmVzaXN0ZW1d&k=resistem [retrieved on 2012-10-09]
- PAUL CHIDOZIE ONYENEKWE: "The composition of the essential oil of dried Nigerian ginger (Zingiber officinale Roscoe)", EUROPEAN FOOD RESEARCH AND TECHNOLOGY, 1 September 1999 (1999-09-01), pages 407-410, XP055039822,

## Description

### TECHNICAL FIELD

The present invention relates to a new use of zerumbone molecule and new compositions comprising zerumbone.

The application fields of the invention are ingredients and compositions for the cosmetics, dermo-pharmaceutical and personal care products industries, zerumbone being used for the treatment of the skin and appendages of mammalian, humans or animals.

Zerumbone is a sesquiterpene (3 isoprene units) with a "humulane" type skeleton. His name is the 2E, 6E, 9E-Humulatrien-8-one. Its developped formula is:

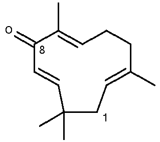

Zerumbone was isolated from roots of *Zingiber zerumbet* (L.) Smith (synonyms: *Amomum zerumbet* L., *Zingiber amaricans* Blume, *Zingiber aromaticum* Val., *Zingiber littorale* Val., *Zingiber zerumbet* (L.) Roscoe), also known as "Ginger Shampoo", "Pinecone Ginger", or "Awapuhi" in Taiwan or "Wild ginger" in China), a plant found in South east Asia. Other plants are said to contain zerumbone as *Alpinia galanga* of Sri Lankan origin or *Syringa pinnafolia*. Zerumbone has anti-inflammatory and anti-radical properties. Effects on cancer in general, especially leukemia, and also effects on the HIV virus are described.

US7588788 describes a nutraceutical composition containing an extract of the roots of *Zingiber zerumbet* (L.) Smith to regulate the immune system and more particularly to prevent and treat allergic disorders.

JP10330243 describes the lightening cosmetic activity of zerumbone extracted from *Zingiber zerumbet* smith.

The present invention aims to propose a new active in particular for the cosmetics, dermo-pharmaceutical and personal care products industries always in demand.

### SUMMARY OF THE INVENTION

To this end, the subject matter of the present invention is the use of zerumbone for a cosmetic non therapeutic topical slimming treatment.

In-vitro evaluation results are given in the below detailed description.

More particularly according to the invention, zerumbone can be used:
- To reduce expansion and/or prevent the development of adipose tissue; and/or
- To reduce the number and volume of adipocytes; and/or
- To activate the combustion of fatty acids; and/or
- as a lipolytic agent; and/or
- For the treatment of cellulite, dimpling of skin, orange peel skin.

*In-vitro* tests described below in the description also show that zerumbone advantageously allows to strengthen the extracellular matrix, including limiting its degradation against various agents. This can advantageously provide better firmness to tissues in addition to slimming.

It is also shown that zerumbone can be used for an anti-aging treatment of adipose tissue, especially via the stimulation of elastin by adipocytes.

Zerumbone may be of synthetic origin or brought in the form of a plant extract containing it, more or less purified, in particular an extract of *Zingiber zerumbet* (L.) Smith., *Alpinia galanga* of Sri Lankan originin or *Syringa pinnafolia*.

The present invention preferably uses zerumbone extracted from *Zingiber zerumbet* (L.) Smith (*zingiberaceae* family and genus *zingiber*).

A plant extract according to the invention can be obtained either directly from the plant or by plant cell culture. Biological material (plant or plant cell culture) is extracted by any method of extraction, eg a conventional aqueous and/or organic solvent (eg alcoholic) extraction or by subcritical or supercritical fluid, microwave or ultra-sounds.

Preferably, according to the invention, subcritical or supercritical fluid extraction is used, in particular by supercritical CO₂, to obtain thus optimal performance and concentration of zerumbone. Liquid CO₂ is an excellent solvent for lipidic products. Inert, it hardly reacts with the molecules extracted and leaves no residue since it is evaporated at the end and captured to be reused.

The extraction can be performed on the whole plant or specific parts of the plant. Preferably according to the invention, the extract is obtained from the roots or rizomes of the plant.

Derivatives of zerumbone are disclosed, including:
- Simple substitutions on the skeleton:
   o OH and OAc in position 1; 5; 8; 14; 15
   o COOH in position 14; 15
   o C=O in position 5; 8
   o CHO in position 14
- Oxidation of the double bonds in epoxide, as for zerumbone oxide or some humulatrien-15,1-elides (Asteriscunolide A to D) having a lactone ring between positions 1 (OH) and 15 (COOH).

The present invention also encompasses compounds close or analogs to zerumbone including:
- The monoenes and dienes (eg Buddledone A (or 2E, 9E-Humuladien-8-one));
- Compounds having a derivative cyclic structure close to the structure of β-Caryophyllene where carbons 2 and 10 are connected with migration of the double bonds in 9-10 in 10-2 and 2-3 to 3-15:

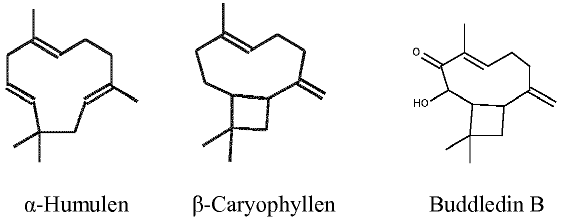

A zerumbone extract according to the invention can be used pure or diluted in a physiologically acceptable medium or matrix. The nature of the medium or matrix is defined according to the properties of the extract, nature of the extraction solvent used for the extract production and also depending on the purpose of the composition formed: single ingredient, galenic form more sophisticated of a consumer final composition.

The present invention thus also proposes a composition characterized in that it comprises, in a physiologically acceptable medium, an effective amount of zerumbone for a slimming treatment. According to other features of the invention, particularly advantageously, zerumbone is combined with a hormetic response agent, mimicking the positive effects of caloric restriction.

This type of agent activates a set of genes including Sirtuins 1, has a strong anti-oxidant power (DPPH, O2°-, ROS, SOD and catalase increase) and antiglycation.

Sirtuins act in particular of the deacetylation of FOXO factor and avoid it triggers the production of apoptotic proteins. The Sirtuins also stimulate the antioxidant defenses and purifying of the cell and allow the preservation of mitochondrial functions and thus production. This increases survival and stress resistance.

Preferably according to the invention, an extract of *Globularia* is used as a hormetic response agent, in particular of *Globularia cordifolia.* Such an extract is the subject matter of the patent application FR1150739 to which reference can be made.

This extract can be obtained by conventional plant extraction or by *in-vitro* plant cell culture.

The description below gives a preferred example of obtaining by *in-vitro* plant cell culture of an extract of *Globularia cordifolia* and details of its biological activities, which combined with that of zerumbone, lead to a combined slimming action particularly interesting according to the invention.

Among the other hormetic response agents mimicking the positive effects of caloric restriction that can also be used according to the invention, as examples polyphenols and the best known the resveratrol can be also cited. Other polyphenols with similar activity are: butein, piceatannol, isoliquiritigenin and quercetin. Hormetins, such as curcumin, Kinetin (plant growth factor); rosmaric acid, alpha lipoic acid, Q10 coenzyme can also be added.

Advantageously, according to the invention another slimming and/or anti-cellulite active agent can be used in complement and reinforcement of zerumbone.

Preferably according to the invention, a lipolytic compound of the family of methylxanthines, such as caffeine, theophylline, theobromine or aminophylline is used. Preferably according to the invention caffeine is used. These compounds are known for their lipolytic action (favoring the hydrolysis of triglycerides accumulated in adipocytes and their release) and anti-cellulite action, and also for their draining action (promoting the elimination of water) and stimulating action (increasing the metabolic activity of cells).

The caffeine used can be from plant origin.

Therefore, a particularly preferred combination for the implementing of the invention comprises zerumbone, the *Globularia cordifolia* extract and caffeine.

As examples can also be cited: phosphodieterase inhibitor compounds, alpha-2 blocker compounds capable of blocking alpha-2 receptors on the surface of adipocytes, agonists and beta-adrenergic antagonists (eg alverine or a organic or inorganic salt of alverine such alverine citrate), compounds inhibiting the synthesis of LDL or VLDL receptors, inhibitors of the enzymes of fatty acid synthesis, such as acetyl CoA carboxylase or fatty acid synthetase or cerulenin, compounds stimulating beta receptor and/or G proteins, glucose transport blockers, such as serine or rutin, antagonists of neuropeptide Y (NPY) capable of blocking the NPY receptors on the surface of adipocytes, AMPc and its cosmetically acceptable derivatives, activating agents of adenylate cyclase such as forskolin, agents modifying the transport of fatty acids, lipolytic peptides and proteins, such as peptides or proteins derived of parathyroid hormone.

Other examples of lipolytic agents that can be used in the context of the present invention include, without limitation, extracts of plant and marine origin:
- From plant extracts, may be cited: the extract of English Ivy (*Hedera helix*), *Bupleurum chinensis,* arnica (*Arnica Montana* L), rosemary (*Rosmarinus officinalis* N), marigold (*Calendula officinalis*), sage (*Salvia officinalis* L.), ginseng (*Panax ginseng*), ginkgo biloba, St. John's wort (*Hyperycum perforatum*), butcher's broom (*Ruscus aculeatus* L), European meadowsweet (*Filipendula ulmaria* L), big- flowered Jarva tea (*Orthosiphon Stamincus Benth*), algae (*Fucus vesiculosus*), birch (*Betula alba*), cola nuts (*Cola Nipida*), green tea, horse chestnut, bamboo, *Centella asiatica,* heather, fucus, willow, mouse-ear, escine, cangzhu, *chrysanthellum indicum* extracts, extracts of plants of the *Armeniacea* genus, *Atractylodis Platicodon*, *Sinommenum*, *Pharbitidi*, *Flemingia*, *Coleus* extracts such as C. *Forskohlii*, C. *blumei*, C. *esquirolii*, C. *scutellaroides*, C. *xanthantus* and C. *Barbatus* as *Coleus barbatus* root extract, *Ballote*, *Guioa*, *Davallia*, *Terminalia*, *Barringtonia*, *Trema*, *Antirobia*, *Cecropia*, *Argania*, *Dioscorea* rich diosgenin such as *Dioscorea opposita* or mexican or *villosa* extracts. As marine extracts extracts of algae or phytoplankton such as an extract of *Laminaria digitata* or rhodysterol can be cited.

According to the invention may also be used in conjunction with zerumbone combinations of anti-cellulite and slimming products marketed by Sederma Vexel™, Coaxel™, Cyclolipase™, Pleurimincyl™, Lipocare™ , Redulite™ and Unislim™.

Other complementary actives can also be used according to the invention chosen among: active acting on the microcirculation (vasculoprotective or vasodilator) such as flavonoids, ruscogenins, esculosides natural or synthetic, escin, nicotinates, heperidin methyl chalcone, ruscus (petit houx), essential oils of lavender or rosemary, extracts of *Ammi visnaga*; firming and/or anti-glycant actives such as extracts of *Centella asiatica* and *Siegesbeckia*, silicon, amadorine, ergothionein and its derivatives, hydroxystilbens and their derivatives, including resveratrol, plant extracts of the *Ericaceae* family, including bilberry (*Vaccinium angustifollium*), vitamin C and its derivatives, retinol and its derivatives .

According to other features, a composition according to the invention can incorporate one or more other active ingredients that may for example be selected from the lightening, anti-redness, UV sunscreens, moisturizers, humectants, exfoliating, anti-aging, anti-wrinkles, volumizing, improving elastic properties, anti-acne, anti-inflammatory, anti-oxidant, anti-radical, propigmenting or depigmenting, depilatories, anti-regrowth, or promoting hair growth, peptides, vitamins active ingredients, etc.. These active ingredients can be obtained from plant materials, such as plant extracts or plant cell culture products or by fermentation.

More specifically, in a cosmetic composition, the extract according to the invention can be combined with at least one of the compounds selected from the compounds of B3 vitamin, the compounds as niacinamide or tocopherol, retinoid compounds, such as retinol, hexamidine, α-lipoic acid, resveratrol or DHEA, peptides, including Pal-KT, Valyl-Tryptophane (VW), N-acetyl-Tyr-Arg-O-hexadecyl ester, Pal-VGVAPG (SEQ ID NO: 1), Pal-KTTKS (SEQ ID NO: 2), Pal-GHK, Pal-KMO2K and Pal-GQPR (SEQ ID NO: 3), which are widely used active ingredients in topical cosmetic compositions.

Preferably according to the invention for a slimming use it can be proposed:
a moisturizing active, an active that can bring a feeling of freshness, an active further improving skin tone, muscles, a draining, soothing, anti-hair regrowth, anti-oxidant active, an active acting on feelings of heaviness, anti-oedemas and/or stretch marks.

The present invention also encompasses a cosmetic topical composition comprising, in a physiologically acceptable medium, zerumbone and at least one compound selected from a hormetic response agent and a lipolytic agent.

Preferably, the composition comprises zerumbone, a hormetic response agent and a lipolytic agent. Moreover, preferably:
- The hormetic response agent is a *Globularia cordifolia* extract, preferably obtained by *in vitro* plant cell culture; and/or
- The lipolytic active agent is from the methylxanthines family, in particular caffeine, theophylline, theobromine and aminophylline, and preferably caffeine, and/or
- zerumbone is extracted from *Zingiber zerumbet* (L.) Smith.

A particularly preferred composition according to the invention, as mentioned above, comprises zerumbone, a *Globularia cordifolia* extract and caffeine.

The present invention encompasses the use of a composition thus recited for a general non therapeutical cosmetic topical treatment, including a slimming treatment, as explained above.

The present invention will be better understood in the light of the following description.

The following examples illustrate the invention without limiting it to these applications.

### DETAILED DESCRIPTION

### Composition preparation for implementing the present invention

The expression "physiologically acceptable medium" means according to the present invention, without limitation, an aqueous or hydroalcoholic solution, a water-in-oil emulsion, an oil-in-water emulsion, a micro-emulsion, an aqueous gel, an anhydrous gel, a serum, a dispersion of vesicles or a powder.

"Physiologically acceptable" means that the compositions are suitable for topical use, in contact with mucous membranes, nails, scalp, hairs and skin of mammals, particularly human, without risk of toxicity, incompatibility, instability, allergic response, and others.

This "physiologically acceptable medium" forms what is commonly called the excipient of the composition.

The effective amount of zerumbone according to the invention, that is to say its dosage, depends on the destination of the composition. It depends on various factors, such as the age, the condition of the patient, the severity of the disorder or disease and the administration mode. An effective amount means a non toxic amount enough to achieve the desired effect.

In a cosmetic composition for the implementing of the present invention, zerumbone, to be present in an effective amount, is generally present in an amount ranging from 0,000001% et 15% based on the total weight of the composition, preferably ranging from 0.00001% et 10%, depending on the destination of the composition and the more or less pronounced desired effect.

All percentages and ratios used herein are by weight of the total composition and all measurements are made at 25°C unless it is otherwise specified.

The choice of the excipient of the composition is made according to the constraints of the zerumbone or the extract containing it (stability, solubility, etc.), and if necessary according to the intended galenic form envisaged afterwards.

Zerumbone is soluble in particular in oil and in an alcohol. It can be incorporated in a composition by means of physiologically acceptable conventional solubilizers, for example and without limiting to this list: ethanol, propanol, isopropanol, propylene glycol, glycerin, butylene glycol, or polyethylene glycol or any combination thereof. It may also be interesting to solubilize the extract using emulsifiers.

A zerumbone extract according to the invention can be used pure or diluted.

All galenic forms that can contain zerumbone and additional ingredients if present, in particular the extract of *Globularia cordifolia* and/or caffeine, can be used, i.e. solution, emulsion, dispersion, suspension, onguent, cream, lotion, milk, ointment, gel, paste, powder, anhydrous preparation (for example oil for skick, "roll-on"), foam, essence, serum, spray, sprayable formulation, brushable, patch, adhesive material, individually or as a premix or vehicled individually or as a premix in a bound form, incorporated or adsorbed in vectors such as macro-, micro-, or nanocapsules, macro-, micro- or, nanospheres, liposomes, oleosomes or chylomicrons, macro-, micro-, or nanoparticles or macro-, micro or nanosponges, micro- or nanoemulsions, or adsorbed on organic polymer powders, talcs, bentonites, spores or exines, and other inorganic or organic supports, etc. The composition may be incorporated onto a non-woven or woven material, with natural or synthetic fibers, wool, or any material intended to come into contact with skin and that can be used in clothing, including tights and socks, shorty, day or night underwear, tissues, handkerchiefs or fabric to exert its cosmetic effect via the contact skin/textile and enable continuous topical delivery (cosmetic-textiles).

According to the invention, it is thus also proposed a woven or non-woven fabric comprising zerumbone for use in a non therapeutical cosmetic topical slimming treatement.

The galenical formulations can enter in different product ranges for personal care and/or beauty products including skin care, cleaning, makeup, cleansing, sunscreen, artificial tanning, pre-shave, shaving or aftershave, moisturizer, humectant, emollient, conditioning, exfoliating, astringent, depilatories or antiperspirant, deodorisant, deodorant, etc.

The CTFA ("International cosmetic ingredient dictionary & handbook" (13th Ed. 2010) published by the "Cosmetic, Toiletry, and Fragrance Association, Inc.", Washington, D.C.) describes a non-limited wide variety of cosmetic and pharmaceutical ingredients conventionally used in the skin care industry that can be used as additional ingredients in the compositions for the present invention, as long as they are physically and chemically compatible with the other ingredients of the composition and especially with the active ingredients of the present invention. Also the nature of these additional ingredients should not unacceptably alter the benefits of the active ingredient of the invention. These additional ingredients can be synthetic or natural such as plants extracts, or come from a bio-fermentation process.

Further skin care actives that are particularly useful combined with the composition can be found in Sederma's commercial literature and on the website www.sederma.fr.

Commercially available actives widely used in cosmetic compositions can also be mentionned as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (commercial name of the acetyl hexapeptide-3 from Lipotec), spilanthol or an *Acmella oleracea* extract known under the trade name Gatuline Expression™, a *Boswellia serrata* extract known under the trade name Boswellin™, Deepaline PVB™ (Seppic), Syn-AKE™ (Pentapharm), Ameliox™, Bioxilift™ (Silab), Juvinity™ (Sederma), Revidrat™ (Sederma), or mixture thereof.

Among plant extracts which can be combined with zerumbone according to the invention, there may more particularly be mentioned extracts of Ivy, in particular English Ivy (*Hedera Helix*), of *Bupleurum chinensis*, of *Bupleurum Falcatum,* of arnica (*Arnica Montana* L), of rosemary (*Rosmarinus officinalis* N), of marigold (*Calendula officinalis*), of sage (*Salvia officinalis* L), of ginseng (*Panax ginseng*), of ginko biloba, of St.-John's-Wort (*Hyperycum Perforatum*), of butcher's-broom (*Ruscus aculeatus* L), of European meadowsweet (*Filipendula ulmaria* L), of big-flowered Jarva tea (*Orthosiphon Stamincus Benth*), of algae (*Fucus Vesiculosus*), of birch (*Betula alba*), of green tea, of cola nuts (*Cola Nipida*), of horse-chestnut, of bamboo, of *Centella asiatica,* of heather, of fucus, of willow, of mouse-ear, of escine, of cangzhu, of *chrysanthellum indicum*, of the plants of the *Armeniacea* genus, *Atractylodis Platicodon*, *Sinnomenum*, *Pharbitidis*, *Flemingia,* of *Coleus* such as C. *Forskohlii*, C. *blumei*, C. *esquirolii*, C. *scutellaroides*, C. *xanthantus* and C. *Barbatus*, such as the extract of root of *Coleus barbatus,* extracts of *Ballote*, of *Guioa*, of *Davallia*, of *Terminalia*, of *Barringtonia*, of *Trema*, of *antirobia*, *cecropia*, *argania*, *dioscoreae* such as *Dioscorea opposita* or Mexican, extracts of *Ammi visnaga,* of *Siegesbeckia*, in particular *Siegesbeckia orientalis*, plant extracts of the family of *Ericaceae*, in particular bilberry extracts (*Vaccinium angustifollium*) or *Arctostaphylos uva ursi*, *aloe vera*, plant containing sterols (e.g., phytosterol), Manjistha (extracted from plants of the genus *Rubia,* particularly *Rubia Cordifolia*), and Guggal (extracted from plants of the genus *Commiphora*, particularly *Commiphora Mukul*), kola extract, chamomile, red clover extract, *Piper methysticum* extract *(Kava Kava™* from Sederma), *Bacopa monieri* extract (Bacocalmine™ from Sederma) and sea whip extract, extracts of *Glycyrrhiza glabra*, of mulberry, of *melaleuca* (tea tree), of *Larrea divaricata*, of *Rabdosia rubescens*, of *Euglena gracilis*, of *Fibraurea recisa Hirudinea*, of *Chaparral Sorghum*, of sun flower extract, of *Enantia chlorantha*, of Mitracarpe of *Spermacocea* genus, of *Buchu barosma*, of *Lawsonia inermis* L., of *Adiantium Capillus-Veneris* L., of *Chelidonium majus,* of *Luffa cylindrica*, of Japanese Mandarin (*Citrus reticulata Blanco* var. unshiu), of *Camelia sinensis*, of *Imperata cylindrica*, of *Glaucium Flavum,* of *Cupressus Sempervirens*, of *Polygonatum multiflorum,* of *loveyly hemsleya*, of *Sambucus Nigra,* of *Phaseolus lunatus,* of *Centaurium,* of *Macrocystis Pyrifera*, of *Turnera Diffusa*, of *Anemarrhena asphodeloides*, of *Portulaca pilosa*, of *Humulus lupulus*, of *Coffea Arabica*, *of Ilex Paraguariensis*, of *Oxydendron arboreum* or of *Ulva Lactuca.* The compositions of the present invention may include peptides, including, without limitation, the di-, tri-, tetra-, penta-and hexapeptides and their derivatives. According to a particular embodiment, the concentration of the additional peptide, in the composition, ranges from 1x10-7% and 20%, preferably from 1x10-6% and 10%, preferably between 1x10-5% and 5%, by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides which are found in nature, and/or are commercially available.

Suitable dipeptides for use herein include but are not limited to carnosine (beta-AH), YR, VW, NF, DF, KT, KC, CK, KP, KK or TT. Suitable tripeptides for use herein include, but are not limited to RKR, HGG, GHK, GKH, GGH, GHG, KFK, GKH, KPK, KMOK, KMO2K or KAvaK. Suitable tetrapeptides for use herein include but are not limited to RSRK (SEQ ID NO: 4), GQPR (SEQ ID NO: 5) or KTFK (SEQ ID NO: 6). Suitable pentapeptides include, but are not limited to KTTKS (SEQ ID NO: 7). Suitable hexapeptides include but are not limited to GKTTKS (SEQ ID NO: 8), VGVAPG (SEQ ID NO: 9).

Other suitable peptides for use herein include, but are not limited to lipophilic derivatives of peptides, preferably palmitoyl derivatives, and metal complexes as aforementioned (e.g. copper complex of the tripeptide HGG). Preferred dipeptide derivatives include N-Palmitoyl-beta-Ala-His, N-Acetyl-Tyr-Arg-hexadecylester (Calmosensine™, Idealift™ from Sederma). Preferred tripeptide derivatives include in particular N-Palmitoyl-Gly-Lys-His and N-Palmitoyl-Gly-His-Lys, (Pal-GKH and Pal-GHK from Sederma), the copper derivative of HGG (Lamin™ from Sigma), Lipospondin (N-Elaidoyl-KFK) and its analogs of conservative substitution, N-Acetyl-RKR-NH2 (Peptide CK+), N-Biot-GHK (from Sederma), Pal-KMO2K (Sederma) and derivatives thereof. Suitable tetrapeptide derivatives for use according to the present invention include, but are not limited to, N-palmitoyl-GQPR from Sederma (SEQ ID NO: 3), suitable pentapeptide derivatives for use herein include, but are not limited to, N-Palmitoyl-KTTKS (SEQ ID NO: 2) (available as Matrixyl™ from Sederma), N-Palmitoyl-Tyr-Gly-Gly-Phe-X (SEQ ID NO: 10) with X Met or Leu or mixtures thereof. Suitable hexapeptide derivatives for use herein include, but are not limited to, N-Palmitoyl-VGVAPG (SEQ ID NO: 1) and derivatives thereof. The mixture of Pal-GHK and Pal-GQPR (SEQ ID NO: 3) (Matrixyl™ 3000, Sederma) can also be mentioned.

The preferred compositions commercially available containing a peptide or a derivative include Biopeptide-CL™, Maxilip™, Biobustyl™, Procapil™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Palmitoyl-GQPR (SEQ ID NO: 3) and carrier, proposed by Sederma.

The following commercially available peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (Infinitec Activos), Cytokinol™LS (Laboratoires Serobiologiques/Cognis), Kollaren™, IP2000™ or Meliprene™ (lnstitut Européen de Biologie Cellulaire), Neutrazen™ (Innovations), ECM-Protect™ (Atrium Innovations), Timp-Peptide™ or ECM Moduline™ (Infinitec Activos).

### Cosmetic treatment method

The present invention also provides a slimming cosmetic method, comprising topically applying to the skin and/or its appendages of a subject in need thereof an effective amount of a composition comprising zerumbone according to the invention and as recited in the present specification.

The invention composition can incorporate additional ingredients advantageous according to the invention as disclosed above for the cosmetic use, i.e. a *Gobularia* extract, in particular of *Globularia cordifolia,* preferably obtained from in vitro plant cell culture, and/or an aditionnal slimming ingredient, promoting lipolysis, in particular of the methylxanthine family, such as caffeine.

The composition according to the invention may be applied locally on areas of the face or the body. One of the major advantages of the present invention resides in the ability whenever necessary or desirable to be able to apply local selective "gentle" treatments through this topical, non-invasive method of application.

The topical composition is preferably applied once daily for a period of at least a week, but it can be applied during periods of 2, 4, 8 or 12 weeks. For indication, for a cosmetic face treatment, the European standard dosage of a cream is 2.72 mg/cm²/day/person and for a cosmetic body treatment the European standard dosage of a lotion is 0.5 mg/cm²/day/person.

According to other features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising zerumbone, and in a second compartment a composition containing another active ingredient (in particular the *Globularia cordifolia* extract and/or caffeine) and/or excipient, the compositions contained in the said first and second compartments in this case being considered to be a combination composition for simultaneous, separate or stepwise use in time, particularly in one of the treatment methods recited above.

The first compartment may also contain a composition comprising a combination according to the invention of zerumbone, *Globularia* extract and caffeine, forming a unique ingredient and the second compartment an excipient.

The treatment method of the invention comprising the topical application of an effective amount of zerumbone is particularly suitable:
- For reducing expansion and/or preventing the development of adipose tissue; and/or
- For reducing the number and volume of adipocytes; and/or
- For activating the combustion of fatty acids; and/or
- As a lipolytic agent; and/or
- For the treatment of cellulite, dimpling of skin, orange peel skin.

The treatment method of the invention is also particularly suitable for an anti-aging treatment of adipose tissue, in particular via the stimulation of elastin by the adipocytes.

### A) Example for obtaining a zerumbone extract

Plant: *Zingiber zerumbet* (L.) Smith.
Part of the plant: dried and crushed roots (rizomes).
Protocol: CO₂ subcritical or supercritical extraction.
Extraction pressure: 70-1000 bars, preferably 70-350 bars.
Temperature: 10-100°C, preferably 20-70°C.
Duration: 24 to 48 hrs.
Purification (filtration, centrifugation, crystallization).

### B) Example of obtaining a Globularia extract by undifferentiated cell culture of Globularia cordifolia

### 1) Creation of a cell line

*Globularia cordifolia* plants were obtained from seeds.

Leaves are collected, and thereafter cleaned and rinsed with water, and decontaminated and further rinsed.

### a) Step of induction of undifferentiated cells or callus

Pieces of leaves are cut and placed on the surface of a nutrient agar, containing an assimilable carbon source, a solution of micro and macro-elements and a combination of adapted hormones and vitamins. This nutrient medium, thanks to its composition, will trigger the production of clusters of undifferentiated cells called callus at the site of healing of the leaf. Composition of the solid nutrient medium used (according to Gamborg): solution of macro-elements and micro-elements, saccharose, agar plant, plant hormones and vitamins.

### pH is adjusted between 5.5 and 6.

This medium will be thereafter referred to the "basic medium".

This induction step lasts 1 to 4 months. Culture is made at 25°C in the dark.

### b) Stabilization and selection step of the cultures in agar medium.

Callus stabilization is obtained after successive transplantations, every 3 weeks, on fresh medium.

Stable and homogeneous cultures are thus obtained thanks to their phenotypic characteristics (color, friability, proliferation).

The selection is to choose the best callus cultures for the transfer in liquid medium, among the tens initiated earlier. The selected lines are characterized by high growth, a soft and crumbly texture, uniform color, good dispersion in liquid medium and stability of these parameters during the subcultures.

This step lasts from 6 months to 1 year.

### c) transfer in liquid medium and optimization of the production of secondary metabolites

The selected lines are transferred in liquid medium in order to optimize in an initial stage their growth parameters.

Cell growth and appreciation of the different phases (latence, exponential, stationary) are assessed by regular measurement of the percentage volume occupied by the culture cells or "packed cell volume" (PCV).

Different suspensions from the callus are placed on an orbital shaker, agitated at 110 rpm in the dark at 25°C (in Erlenmeyer flasks each containing 100 ml of medium and 10 grams homogeneous texture, color and growth callus). Several media and hormone combinations are tested and compared in order to optimize the growth curve. The optimization of the initial density of the inoculum is also seeked for reducing the lag phase and thus the global duration of culture. Finaly, the chosen media is the basic medium to have a liquid medium. Furthermore, the selected initial density for the inoculum corresponds to a PCV of 10%.

The next step is to determine the optimal environmental conditions to direct the cellular metabolism toward the production of secondary metabolites at the end of the growth phase of the culture. These secondary metabolites and the other components of the extract medium as well, will participate in the activity of the extract obtained at the end.

Thus, in the medium, a high proportion of polar molecules were identified, including sugars, amino acids, primary metabolites, cinnamic acid and caffeic derivatives, and mixture of phenylethanoid glycosides. The phenylethanoid glycosides were chosen as indicators of secondary metabolites biomass production, their total dosage being easily achieved by the method of Arnow, a spectrophotometric method based on the oxidation of total polyphenols in alkaline medium by sodium nitrite and sodium molybdate (reading at 510 nm). Other classes of molecules are assumed to be present in the medium, such as flavonoids or iridoids but could not be identified. It is obvious that all of these molecules will be responsible for the activity of the final extract.

The elicitation chosen according to the invention to lead to the production of secondary metabolites is then to place the cells in medium depleted of sucrose and macronutrients, preferably in the same proportions (ratio 1:1).

This transfer phase in liquid medium and optimization of the parameters of growth and production can last from 6 months to 1 year.

After this step, a line giving the best results was selected.

It was subjected to a deposit with DSMZ (Deutsche Sammlung von Mikroorganismen und Zellkuturen GmbH) under the reference DSM25009.

### 2) Extract obtaining

### a) Production of pre-cultures in bioreactors

Successive pre-cultures of the cell line selected of 1-liter, then 5 liter, and then 25 liter and finally 100 liter are realized. Each is seeded at 10% PCV and then grown to 50% of PCV. The cell biomass thus produced is used to inoculate the larger size culture. This stage can last from 1 to 3 months.

### b) Transfer to bioreactor comprising the elicitation phase

A single use bag bioreactor (600 liters of total volume and 300 liters of total culture volume) is then seeded between 5 and 20% PVC.

The bioreactor is in particular equipped with:
- A rocker system for cell agitation and oxygenation, and for culture temperature regulation, preferably selected because being more suitable with regard to the fragility of plant cells compare to shearing of a conventional bioreactor;
- A control tower equipped with different sensors (oxygen, pH and temperature) for the follow-up and the regulation of the culture steps;
- A single-use sterile bag for the cell culture, equipped with different sensors (pH, oxygen), a filter for gas supply and exchange systems for sampling or inoculating sterile fluids.

After 10-15 days of culture in the bioreactor, the culture reaches a PCV of about 30 to 45%. The deficient medium is then added to trigger the production of secondary metabolites, including here phenylethanoid glycosides. The addition of fresh medium is 10 to 50% of the final culture volume. After 3-5 days, the culture was stopped at 35-50% of PCV, the phenylethanoid glycosides then being around 2 grams per liter in the culture.

This entire phase in bioreactor lasts 2 to 3 weeks.

### c) Recovery of the extract

The aim of this subsequent step is to produce an aqueous and clarified extract.

A cell lysis is achieved, leading to the release of secondary metabolites in the liquid phase. This lysis is carried out by acidification. A cascade filtration is then carried out, the first in-depth, followed by sterile filtration, to obtain the aqueous extract of the invention. This step is intended to stabilize the extract.

The extract obtained this way by plant cell culture is clear, colorless to pale yellow, to the contrary of the "classic" plant extract which is bright yellow, which is an advantage for a direct subsequent formulation of the extract in a cosmetic ingredient or other.

### Variants of this process exist, including:

1) For the extraction of the molecules of interest:
   - The cell lysis step can be performed by biomass thermal treatment, for example by injecting steam into the biomass. It can also be made by grinding the cells with microbeads.
   - After the cell lysis step, it is possible to purify the crude extract, in order to enrich it in molecules of interest. According to this, biomass is first removed by filtration or centrifugation. The resulting clarified liquid phase is then contacted with an adsorbent resin, selected for its affinity with the molecules of interest. This resin is then loaded into a glass chromatography column. The resin is eluted with appropriate mixtures and the elution of the product concentrated.
   - The recovery of the extract according to the invention can be implemented using liposomes.
2) Regarding the elicitation mode of the molecules of interest:
   The elicitation of compounds of interest can also be done by adding microbial fractions to the culture, by adding molecules of biological origin such as, chitosan, methyljasmonate, jasmonic acid, salicylic acid, by adding molecules of non-biological origin such as paclobutrazol, by applying to the culture a temperature variation, a pH variation, and/or an osmotic stress induced by a non-metabolizable sugar, such as mannitol, by using an even more drastic impoverishment of the environment in macro-elements and sugar, by adding to the culture adsorbent resins that in addition to elicitation of the compounds of interest production can trap them.
3) Instead of the cell line creation stage, a cell line according to the invention already prepared and preserved can be used. In this case, the step of creating the cell line is replaced by a conventional step of amplification of undifferentiated plant cells from said cell line existing first in agar and in liquid medium.
   The invention method could also be achieved with the other species of *Globularia.*

### 3) Formulation of an « active ingredient », raw material for the cosmetics industry

The aqueous extract obtained above in B) may preferably be mixed at 20 to 80% with any hydrophilic matrix as a gel, an aqueous buffer, glycerin or other short-chain polyol physiologically acceptable.

For example, and for the achievement of the studies and galenic presented thereafter, an extract up to 33% was preferentially used.

### C) In-vitro evaluation of zerumbone

### 1. Slimming activity

### a- Anti-differentiating effect on adipocytes - anti-storage effets

### • On 3T3-L1 line

Murine 3T3-L1 fibroblasts were seeded and induced to differentiate into adipocytes for 6 days with specific inducers in the presence of the evaluated products. When mature adipocytes were obtained, layers were crushed and intracellular G3PDH (Glyceraldehyde 3-Phosphate Dehydrogenase) activity is measured. A cell quantification test (Hoechst) on the same extracts complements the assay.

**Table 1:**

| **G3PDH- 3T3L1** | | **[G3PDH] mU/ 10⁶ cell** | **% Var / control** |
|---|---|---|---|
| **Control** | - | 652.6 +/- 130.5 | **Ref** |
| **Zerumbone** | **1ppm** | 304.2 +/- 9.9 | **-53%;** p<0.01 |
| | **3ppm** | 79.3 +/- 43.6 | **-88%;** p<0.01 |
| | **5ppm** | 3.4+/-10.3 | **-99%;** p<0.01 |

Zerumbone strongly inhibits in a dose-dependent manner and without apparent cytotoxicity differentiation of 3T3-L1. This effect reaches a total inhibition of differentiation at 5ppm.

### • On human adipocytes

Human preadipocytes were plated and induced to differentiate at confluence in the presence of a cocktail of specific inducers and the evaluated products. After 10 days, the layers were crushed and intracellular G3PDH activity was assayed. A cell quantification test (Hoechst) on the same extracts complements the assay.

**Table 2:**

| **G3PDH-AH** | | **[G3PDH] mU/ 10⁶ cell** | **% Var / control** |
|---|---|---|---|
| **Control** | - | 175 +/- 9 | Ref |
| **TGFβ1** | **10⁻⁶%** | 29 +/- 4 | 84%; p<0.01 |
| **Zerumbone** | **0.5ppm** | 144 +/- 15 | -18%; p<0.05 |
| | **1ppm** | 106 +/- 14 | -39%; ρ<0.01 |
| | **3ppm** | 30 +/- 2 | -83%; p<0.01 |
| | **5ppm** | 15 +/- 3 | -91%; p<0.01 |

Zerumbone strongly inhibits dose-dependently G3PDH activity and human preadipocytes differentiation to complete inhibition at 5 ppm of extract.

### Dosage of the triglyceride content

Human preadipocytes were plated and induced to differentiate at confluence in the presence of a cocktail of specific inducers and evaluated products. After 10 days, the triglyceride content of the culture was estimated by measuring the released glycerol after the action of a lipase.

**Table 3:**

| **TG-AH** | | **[TG] µ U/ 10⁶ cell** | **% Var / control** |
|---|---|---|---|
| **Control** | - | 4181 +/- 304 | Ref |
| **TGFβ1** | **10⁻⁶%** | | -22%; p<0.01 |
| **Zerumbone** | **3ppm** | 3562 +/- 233 | -15%; p<0.05 |
| | **5ppm** | 2966 +/- 128 | -29%; p<0.01 |

A decrease of the triglyceride content after preadipocyte differentiation into adipocytes in the presence of zerumbone is observed.

### b- Lipolytic effects by measuring glycerol

Mature human adipocytes were treated with zerumbone to evaluate daily for 3 days. A daily titration of the released glycerol, reflecting lipolysis, was measured in the medium, before the media were changed in order to monitor the long-term effect.

**Table 4:**

| **Contact time** | **+Zerumbone (6ppm); significance** |
|---|---|
| **1 day** | +72%; *p<0.01* |
| **2 days** | +141%; *p<0.01* |
| **3 days** | +246%; *p<0.01* |

The results show a strong release of glycerol from the second day, the addition of zerumbe the third day further reinforcing this effect.

### • Triglyceride assay

**Table 5: comparison of the intracellula triglyceride quantity found in mature adipocytes**

| **Concentrations** | **Triglycerides (µM/10⁶cell.)** | **Variation (%); significance** |
|---|---|---|
| **Control** | 7847 ± 681 | Ref. |
| **Zerumbone 6ppm** | 4947 ± 236 | -37%; p<0.01 |

These results confirm those previously observed: zerumbone can have a clear lipolytic effect as the triglyceride amount is reduced compared to the negative control.

### c- Adiponectin synthesis

Human preadipocytes were plated and induced to differentiate at confluence in the presence of a cocktail of specific inducers. After 10 days of differentiation, the cells are purged from inducers in their basis medium and left in contact with the products for 3 days in this medium. After contact, the supernatants were collected and the adiponectin amount was measured by ELISA.

**Table 6:**

| **Adiponectin-AH** | | **[Adiponectin] ng/10^{E}6 cell** | **% Var / control** |
|---|---|---|---|
| **Control** | - | 152.7 +/- 14.9 | Ref |
| **Zerumbone** | **2ppm** | 221.4 +/- 24.5 | +45%; p<0.01 |
| **Control** | - | 162.2 +/- 16.0 | Ref |
| **Zerumbone** | **6ppm** | 19.0 +/- 0.6 | +95%; p<0.01 |

A dose-dependent stimulation is observed of adiponectin synthesis was well observed in the presence of the zerumbone extract.

### d- HO-1 synthesis

HO-1 enzymes catalyse the degradation of heme into biliverdin, iron, and carbon monoxide. Its overexpression is associated with a decrease in the size of adipocytes in adipose tissue, an increase of adiponectin secretion and a decrease of IL-6 and TNF secretion and is associated with weight loss.

Human preadipocytes were seeded and induced to differentiate at confluence in the presence of a cocktail of specific inducers and the products to evaluate. After 10 days, the layers were crushed and the HO-1 content was measured in these lysates by ELISA. Furthermore after 10 days of differentiation, matiure adipocytes were placed in contact with the products for 48h and the HO-1 content was also measured in the cellular lysates. In parallel, an estimation of the cell quantification is realized on these two assays (Hoechst test).

**Table 7:**

| | | **Adipocytes in the course of differenciation (10days)** | | **Mature adipocytes (48h)** | |
|---|---|---|---|---|---|
| **HO-1 AH** | | **[HO-1] ng/ 10⁶ cell** | **% Var / control** | **[HO-1] ng/ 10⁶ cell** | **% Var / control** |
| **TGFβ1** | **10⁻⁶%** | - | +45%; p<0.01 | | +117%; p=0.01 |
| **Control** | - | 40908 +/- 10488 | Ref | 29046 +/- 2264 | Ref |
| **Zerumbone** | **3ppm** | 51658 +/- 7985 | +29% p<0.05 | 70229 +/- 10638 | +142% p<0.01 |
| | **5ppm** | 67243 +/- 11428 | +68% p<0.01 | 96700 +/- 4548 | +233% p<0.01 |

A strong stimulation of the synthesis of HO-1 on mature adipocytes in the presence of zerumbone during differentiation was observed.

### e- IL6 synthesis

Human preadipocytes were seeded and induced to differentiate in the presence of a cocktail of specific inducers and the evaluated products. At the end of differentiation (at 7 and 10 days), the IL6 content synthetized by the adipocytes is measured in the culture supernatants by ELISA method. Furthermore, after 10 days of differentiation, mature adipocytes are placed in contact of the products during 72h and the IL6 content is also measured in the cellular supernatants. In parallel, survival estimation was realized on these two assays (Hoechst tests).

**Table 8:**

| | | **Adipocytes in the course of differenciation (10days)** | **Adipocytes after differenciation (3days)** |
|---|---|---|---|
| **IL6** | | % Var / control | % Var / control |
| **TGFβ1** | **10-⁶M** | +246%; p<0.01 | - |
| **Zerumbone** | **3ppm** | -49%; p<0.01 | -33%; p<0.01 |
| | **5ppm** | -50%; p=0.09 | -31%; p<0.01 |

A strong inhibition of IL6 on adipocyte during differentiation and on mature adipocytes in the presence of the zerumbone extract was observed.

### 2. Reinforcing of the extracellular matrix:

MEC strenghtening, in particular by limiting its degradation due to variaous agents, can allow better firmness to tissues in complement of slimming.

### a- Effect on elastin synthesis, under NO stress

Principle: Explants (41-year-old female, abdominal skin) received a topical cream containing the product of the invention (cream according to above example 1 at 18ppm of zerumbone) or a placebo cream (same cream without zerumbone) for two days. At the same time, the culture medium received 18ppm of zerumbone in a liquid form (or its excipient) to potentiate the effects. A NO stress was later applied for 24 hours using an NO donor (NOC-5) integrated in the culture medium. Finally, the product was again applied on the explants and in the culture medium for two days.

The explants were subsequently fixed and elastin fibres were revealed using aVerhoeff stain on 4 µm sections. Counting was done using photos (50 photos/case) by filtering the colours.

**Table 9:**

| **Concentration** | **Elastin (UCA)** | **% Variation; significance** | |
|---|---|---|---|
| **Control without NO** | 6.54 ± 4.5 | Reference 1 | - |
| **Control NO** | 1.18 ± 1.00 | -82%; p<0.01 | Reference 2 |
| **18 ppm of zerumbone** | 2.41 ± 1.40 | - | +104%; p<0.01 |

Results show that the elastin fibres are diminished in number and thickness due to the NO treatment compared to the non stressed control case. The use of zerumbone according to the invention helps maintain the integrity of these fibres thanks to an anti-NO action.

Moreover, a study of normal human fibroblasts (NHF) in culture showed protection of elastin synthesis of +179% (p <0.01) with 5 ppm of the product according to the invention, compared to the control case following the same NO. ELISA test.

### b- Fibromodulin and dermatopontin synthesis

These two molecules although in small amounts in the dermis, play a crucial role in the organization of the extracellular matrix. It is therefore important to preserve and promote their synthesis.

Principle: NHFs were put into contact with the product of the invention for two days (fibromodulin) or three days (dermatopontin). The supernatants were then assayed using ELISA tests. At the same time, the number of cells was quantified using the Hoechst method.

**Table 10:**

| **Concentration** | **Fibromodulin (ng/106cell.)** | **% Variation; significance** | |
|---|---|---|---|
| **Control** | 14.0 ± 2.2 | Reference 1 | - |
| **5 ppm of zerumbone** | 51.4 ± 13.4 | - | +268%; p<0.01 |

**Table 11:**

| **Concentration** | **Dermatopontin (ng/106cell.)** | **% Variation; significance** | |
|---|---|---|---|
| **Control** | 1073 ± 104 | Reference 1 | - |
| **5 ppm of zerumbone** | 2540 ± 361 | - | +137%; p<0.01 |

The synthesis of these two important elements, fibromolulin and dermatopontin, is thus facilitated with zerumbone.

### c- MMP1 and Cathepsin-S inhibition

Principle: NHFs were put into contact with the product of the invention for 24h, and then were radiated with UVA rays to trigger an experimental increase in MMP1 synthesis. The cells once again received the product for 24h and the supernatants were assayed using ELISA tests. At the same time, the number of cells was quantified using the Hoechst method.

**Table 12:**

| **Concentration** | **MMP1 (ng/106cell.)** | **% Variation; significance** | |
|---|---|---|---|
| **Control** | 1406 ± 168 | Reference 1 | - |
| **5 ppm of zerumbone** | 691 ± 225 | - | -51%; p<0.01 |

These results show that zerumbone decreases the synthesis of MMP1 induced by an UVA stress. This inhibition of MMP1 allows strengthening of the ECM.

Principle: NHFs were put into contact with the invention product for 24h then were subjected to NO stress. NO donor, NOC-5, was introduced in the culture medium. At the end of a second 24h contact with the zerumbone extract, a cathepsin-S assay was performed using ELISA. At the same time, the number of cells was quantified using the Hoechst method.

**Table 13:**

| **Concentration** | **Cathepsin-S (pg/106cell.)** | **% Variation; significance** | |
|---|---|---|---|
| **Control** | 405 ± 35 | Reference | - |
| **5 ppm of zerumbone** | 290 ± 40 | - | -34%; p<0.01 |

These results show that the zerumbone decreases the synthesis of cathepsin S induced by nitrosative stress.

### d- Effect of dermal destruction by proteases

Principle: Once prepared, skin explants (40-year-old female, abdominal skin) received an injection of elastase or collagenase that could proteolyse 30 µmol/min of elastin or collagen. In another case, the invention product (titrated at 18ppm of zerumbone) was co-injected with the protease to assess its ability to inhibit the proteolysis of constituents of the dermis, and thus of protection of the dermis. After five days of incubation at 37°C, the result was observed through a confocal microscope and compared to what was obtained in the case without protease at the same depth (60 µm).

In the cases where a collagenase or elastase stress was applied, it is shown very clearly that the dermis is altered (denaturation of the elastin or collagen microfibers) and disorganized in the control but retains its integrity in the presence of a solution containing zerumbone.

Thus these tests show that the zerumbone extract can control the activity and the synthesis of proteases known to modify the structure of the dermis and enable, maintain or protect the synthesis of essential elements of the dermal extracellular matrix.

### D) In-vitro evaluation of an extract of Globularia cordifolia (2% of an extract as obtained in above example B)

### 1- Activity on Sirtuins 1 :

Human keratinocytes were cultured in the presence or absence of the *Globularia cordifolia* extract for 6 days and were then grinded and the intracellular levels of sirtuin-1 were measured using an ELISA method. Results were standardized by parallel measurement of total protein (BCA).

**Table 14:**

| | | **Sirtuin-1 (ng/mg protein)** | **% Variation; significance** |
|---|---|---|---|
| **Keratinocytes; n=5** | Control | 2.10 ± 0.58 | Reference |
| | *Globularia cordifolia* extract | 3.65 ± 0.33 | **+74%;** *p<0.01* |

Positive control Trolox at 500µm: +27%; *p<0.05.*

In parallel, abdominal skin explants (58years female) received a topical application daily for 6 days of a cream containing the *Globularia cordifolia* extract of the invention or its placebo (*ex vivo* test). After this treatment, the explants were prepared and labeled with fluorescent antibodies to quantify sirtuin-1. This quantification uses an image analysis system after photography (50 photos / per case).

**Table 15:**

| | | **Sirtuin-1 (Fluorescence intensity).** | **% Variation; significance** |
|---|---|---|---|
| Skin explants; n=5 | Placebo | 10.94 ± 2.40 | Reference |
| | *Globularia cordifolia* extract | 12.23 ± 2.80 | +12%; *p<0.03* |

Stimulation of Sirtuin-1 also works on normal human fibroblasts.

Normal human fibroblasts (NHF) proliferative at subconfluence were cultured (in 100mm boxes) and contacted with the products 24 hours after seeding, for a period of 72 hours in their growth medium (10% FCS DMEMc). After this first incubation, confluent layers were re-contacted with the products for 48 hours in their growth medium. The layers then undergo extraction prior to assay of the Sirtuin-1 by ELISA in the cell extract. A protein assay (BCA) was realized to allow a weighting of the obtained data.

**Table 16:**

| | | **Sirtuin-1 (ng/mg protein)** | **% Variation; significance** |
|---|---|---|---|
| Fibroblasts; n=5 | Control | 0.658 +/- 0.077 | Reference |
| | *Globularia cordifolia* extract | 1.047 +/- 0.139 | **+59%**; *p*<*0.01* |

### 2- Anti-glycation activity:

The anti-glycation effect of the extract of *Globularia cordifolia* was studied using a protein model, the BSA, and a natural reducing sugar, fructose which glycates, i.e. binds, to the protein in a non-enzymatic manner. The product of this binding is fluorescent and is monitored with a fluorimeter. Results are given below in Table 17.

**Table 17 :**

| | | **Glycation (Fluorescence intensity)** | **% Variation; significance** |
|---|---|---|---|
| BSA protein model; n=4 | Control | 21206 +/- 140 | Reference |
| | *Globularia cordifolia* extract | 7668 +/- 121 | **-64% ;** *p<0.01* |

Positive control aminoguanidine at 0.03%: -85%; *p<0.01.*

### 3- Inhibition of RO formation on FHN

Proliferative normal human fibroblasts (NHF) at subconfluence were seeded and contacted 24 hours after seeding with the products for a period of 72 hours in their growth medium. After this first incubation, the confluent layers were subjected to a 2 hours hydrogen peroxide stress (H₂O₂ at 800µM) in the same medium without the products. After rinsing, the layers were contacted with the products for a recovering period of 48h still in their growth medium. Then the cells were trypsinised, counted and re-seeded (with the medium comprising the actives) and cultured until confluence or contacted with the products to test for 24h in a HBSS buffer without glucose. The cells are loaded with the DCFH-DA (50 microns) probe for 30min without products. Then the cells were again placed in the presence of the products for 2 hours in a culture medium with 5% SVF. A reading of the fluorescence is then performed. The fluorescence is directly proportional to the content of intracellular ROS.

**Table 18:**

| | | **ROS (UFA / million of cells)** | **% Variation; significance** |
|---|---|---|---|
| **Fibroblasts; n=5** | Control | 133812 +/- 8110 | Reference |
| | *Globularia cordifolia* extract | 63528 +/- 4414 | -53%; p<0.01 |

### D) In vitro evaluations of a combination according to the invention comprising zerumbone and caffeine

### a- Lipolytic effects

### • Measurement of the released glycerol

In a first test, mature human adipocytes were treated with the product to evaluate for 3 hours, then titration of the released glycerol, reflecting lipolysis, was measured in the medium.

In a second test, mature human adipocytes were treated with the product to evaluate daily for 3 days. A daily titration of the released glycerol was performed in the media, just before changing of the media in order to monitor the long term effect.

**Table 19: Comparison of the lipolytic effect of caffeine, zerumbone and of a mixture of caffeine and zerumbone on hypertrophied adipocytes in monolayer (n=4) at short and long term.**

| **Contact time** | **Caffeine 300ppm** | **Zerumbone (6ppm)** | **200ppm of caffeine + 4ppm of zerumbone** | **300ppm of caffeine + 6ppm of zerumbone** |
|---|---|---|---|---|
| **3h** | +171% | 0% | ND | ND |
| **1 day** | +200% | +72% | +133% | +398% |
| **2 days** | +34% | +141% | +154% | +533% |
| **3 days** | +18% | +246% | +318% | +392% |

| | | | | |
|---|---|---|---|---|
| *All values are significant (at leastp<0.05) versus negative control.* | | | | |

These results show the benefits and the synergy of the association of zerumbone and caffeine in order to promote short- and long-term lipolysis in the mature adipocyte. Caffeine has a clear short-term effect (+171% in the initial hours). Although, adding caffeine daily alone does not maintain the effect over several days and the adipocyte ceases to respond. On the other hand, contact with the mixture of zerumbone and caffeine shows that the adipocyte release large amounts of glycerol from day 2 and that this effect is further increased on the 3rd day. By combining the two substances, lipolysis is very extensively stimulated from day 1 (+398% with the mixture comprising 6ppm of zerumbone), more than is seen by adding the result of zerumbone alone (+72% with the mixture comprising 6ppm a zerumbone) and of cafferine alone (+200% with the mixture comprising 6ppm of zerumbone). A synergy is thus well observed thanks to the combination. This stimulation, with the mixture comprising 6ppm of zerumbone, further increases on day 2 (+533%). In addition, at day 3 of contact, this stimulation is still important and near the two firsts (+392%).

### • Triglyceride titration

**Table 20: Comparison of the intracellular triglyceride content found in the mature adipocytes; compared effect of zerumbone, caffeine and of a mixture of zerumbone and caffeine.**

| **Concentrations** | **Triglycerides (µM/10⁶ cell.)** | **Variation (%); significance** |
|---|---|---|
| **Control** | 7847 ± 681 | Ref. |
| **Zerumbone 6ppm** | 4947 ± 236 | -37%; *p<0.01* |
| **Caffeine 300ppm** | 7120 ± 542 | -9%; *dns* |
| **200ppm of caffeine + 4ppm of zerumbone** | 5940 ± 309 | -24%; *p<0.01* |
| **300ppm of caffeine + 6ppm of zerumbone** | 4129 ± 396 | -47%; *p<0.01* |

These results confirm those previously observed: the association of zerumbone and caffeine can bring a net lipolytic effect as in the two association examples of the table the triglyceride amount is reduced compared to negative control.

### E) In vitro evaluation of a combinaion according to the invention comprising zerumbone, a Globularia cordifolia extract (3% of the extract as obtained in above example B) and caffeine

The effects disclosed above assigned to zerumbone and/or to a combination of zerumbone+caffeine are also found for a mixture of zerumbone+caffeine+*Globularia cordifolia* extract. The following additional effects were also further demonstrated for the mixture zerumbone+caffeine+*Globularia cordifolia* extract.

### a- Desnutrin

Desnutrine is an enzyme located on the surface of lipid droplets of adipocytes. It promotes lipolysis by cutting the stored triglycerides. It increases the fatty acid oxidation activity thus released and energy production by the mitochondria of the adipocyte.

Hypertrophied human adipocytes in culture were contacted with the composition to test for 7 days. The desnutrin amount was then assayed by the ELISA method after cellular extraction. Protein BCA assay allowed to know the amount of cells.

**Table 21: Desnutrin variation (expressed in enzymatic units) in mature adipocytes matures; effect of the combination (zerumbone+caffeine+Globularia cordifolia) (n=3) after 7 days:**

| **Concentrations** | **Desnutrin (µUI/µg protein)** | **Variation (%); significance** |
|---|---|---|
| **Control** | 13.6 ± 4.00 | Ref. |
| **Association (zerumbone 6ppm+caffeine 300ppm+*Globularia cordifolia* 3%)** | 27.6 ± 3.20 | +104%; *p*<*0.01* |

These results show a net stimulation of the content of desnutrin in the mature adipocyte in contact with the association (zerumbone+caffeine+*Globularia cordifolia*).

### b- Harmful effects of trans FA (trans fatty acids) on adipocytes

The organism draws in its food the fatty acids (FA) required for its functioning. Excess sugar and lipids of food will lead to production and storage of triglycerides in the adipocyte. Storage of the different food FA in adipose tissue is selective depending on their quantity and their molecular structure. Polyunsaturated FA limits the hypertrophy of adipose tissue by directing the energy ingested towards its use rather than towards its storage. On the contrary, saturated and mono-unsaturated FA facilitate this hypertrophy via inflammatory phenomena. The saturated and unsaturated *trans* fatty acids (*trans* FA) are fatty acids among the most harmful, in particular because they reduce the fluidity of cellular membranes and disorder the answer of the adipocyte to insulin. These linear fatty acids are found in animal fats. *Trans* FA carry a carbon double bond that deforms them in the trans configuration and that is often produced in the industrial manufacture of oils of vegetable origin. Besides their role in cell membranes, *trans* FA, inherently unstable, accelerate aging of adipose tissue via stimulation of MAP-kinases and AP-1 which triggers the production of proinflammatory cytokines and macrophage attraction. Under these conditions, macrophages greatly increase their production of cytokines (IL-6, MIF, MCP-1 ...), proteases (MMPs and cathepsins), NO and O2°-.

All this damages cells and their extracellular matrices, this effect being enhanced by the production of reactive oxygen species or NO (peroxynitrite) that attack the lipid and protein structures. This contributes to reduce the self-assembly of elastin and lead to the formation of structures less effective while reducing anchor links between matrix proteins and its annexes.

An experimental mixture of *trans* FA was prepared after including rumenic, vaccinic, elaidic and arachidonic acids. This mixture was applied to the cells to verify the adverse effects of these adipotoxines.

Three methods were used.
- Human pre-adipocytes in monolayer were induced to differentiate in contact with the *trans* FA solution with or without the combination of zerumbone+caffeine+*Globularia cordifolia* for 7 days. Once mature adipocytes had been obtained, their intracellular triglycerides were measured to compare differentiation. Cell numbers were measured by the Hoechst method.
- Mature adipocytes were placed in contact for 7 days with a solution of *trans* FA with or without the combination of zerumbone+caffeine+*Globularia cordifolia.* Once mature adipocytes had been obtained, their intracellular triglycerides were measured to compare storage. Cell numbers were measured by the Hoechst method.
- Mature adipocytes were pre-incubated for 3 days with or without the combination of zerumbone+caffeine+*Globularia cordifolia* then were placed in contact with the *trans* FA solution for 24 hours. IL-6 was measured by Elisa in the culture medium. Cell numbers were measured by the Hoechst method.

As shown by the results in the following table, the harmful effects of the *trans* FA were confirmed. In culture, these increased differentiation by +66% compared to the control; triglyceride storage was increased in the mature adipocyte by +99% and IL-6 production by the cells was increased by +41%.

**Table 22: Protection from the harmful effects of the trans FA by the combination of zerumbone+caffeine+Globularia cordifolia; (n=4)**

| **With *trans* FA** | **Differenciation Pre-adipocytes → Adipocytes *(1)*** | **Variation (%); significance** | **Storage *(2)*** | **Variation (%); significance** | **IL-6 *(3)*** | **Variation (%); significance** |
|---|---|---|---|---|---|---|
| **Control** | 1806 ± 209 | Ref. | 3038 ± 136 | Ref. | 6684 ± 666 | Ref. |
| **Association (6ppm zerumbone+300ppm caffeine +*Globularia cordifolia* 3%)** | 1469 ± 109 | -19%; *p*<*0.05* | 1809 ± 42 | -40%; *p*<*0.01* | 3094 ± 375 | -54%; *p*<*0.01* |

| | | | | | | |
|---|---|---|---|---|---|---|
| *(1): trans FA=50µM*; *(2* & *3: trans FA=120 µM;* (*1* & *2*)*: in µM triglycerides*/*10° cell. (3): in pg*/*10⁶ cell* | | | | | | |

The combination (zerumbone+caffeine+*Globularia cordifolia* 2%) reduced differentiation compared to the *trans* fatty acids model by 19%; lipid overload by 40% and IL-6 production by 54%.

### c- Anti-ageing effects of adipose tissue

### • Elastin synthesis

Mature human adipocytes were placed in contact with the combination *(zerumbone+caffeine+Globularia cordifolia)* for 4 days than elastin synthesis was assayed by Elisa. The number of cells was counted by the Hoechst test to homogenize the data.

**Table 23: Variation of elastin synthesis by mature adipocytes; effect of the association (zerumbone +caffeine+Globularia cordifolia) (n=3) after 4 days.**

| **Concentration** | **Elastin (ng/µg protein)** | **Variation (%); significance** |
|---|---|---|
| **Control** | 58.2 ± 1.9 | Ref. |
| **4ppm of zerumbone + 200 ppm of caffeine + 3% of *Globularia cordifolia*)** | 79.7 ± 12.3 | +37%; *p*<*0.05* |
| **6ppm of zerumbone + 300ppm of caffeine + 3% of *Globularia cordifolia* extract)** | 152.2 ± 11.2 | +162%; *p*<*0.01* |

These results, with dose-effect, show a clear stimulation of the amount of elastin by the mature adipocyte in contact with the association zerumbone, caffeine and *Globularia cordifolia* extract. Stimulating of elastin synthesis increases the tissue surrounding the adipocytes and helps fight against the damaging effects of macrophage proteases.

Using a mixture zerumbone, caffeine and *Globularia cordifolia* extract according to the invention can therefore help fighting against aging of adipose tissue by improving its matrix, in particular *via* stimulation of elastin synthesis, which maintains the adipocytes.

### • Attraction and homeostasis of macrophages

- Mature adipocytes (3T3-L1) were cultured in the presence of a mixture zerumbone, caffeine and *Globularia cordifolia* extract for 5 days. At this stage, cell line macrophages (RAW 264.7) cultured in small baskets were then placed above the adipocyte culture but not in contact with the adipocytes. This widely used system is used to count the macrophages attracted by the adipocyte messengers through the crucible at 24 hours.
- Mature human adipocytes were placed in contact with the mixture zerumbone, caffeine and *Globularia cordifolia* extract for 24 hours. Adiponectin, which is known to reduce the macrophage excited state and inhibit their attraction, was assayed by Elisa. The number of cells was counted by the Hoechst test.
- Mature human adipocytes were placed in contact with the mixture zerumbone, caffeine and *Globularia cordifolia* extract for 3 days (IL-6) or 7 days (MCP-1 and MIF). Assays were performed by Elisa. Cells numbers were counted by the Hoechst method.

**Table 24: Variation in macrophage attraction by mature adipocytes; effect of the combination (zerumbone+caffeine+Globularia cordifolia extract) (n=4) after 24h.**

| **Concentrations** | **Number of attracted macrophages (x10³ cell.)** | **Variation (%); significance** |
|---|---|---|
| **Control** | 140 ± 29 | Ref. |
| **6ppm of zerumbone+300ppm of caffeine+*3%* of *Globularia cordifolia* extract)** | 47 ± 10 | -66%; *p<0.01* |

**Table 25: Variation of adiponectin synthesis (macrophagic attraction inhibitor) by mature adipocytes; effect of the combination (zerumbone+caffeine+Globularia cordifolia extract) (n=4) after 24h**

| **Concentrations** | **Adiponectin (pg/10⁶ cell.)** | **Variation (%); significance** |
|---|---|---|
| **Control** | 162 ± 16 | Ref. |
| **6ppm of zerumbone+300ppm of caffeine+*3%* of *Globularia cordifolia* extract)** | 281 ± 31 | +73%;*p<0.01* |

**Table 26: Variation of the synthesis of the macrophage attractive and pre-inflammatory molecules by mature adipocytes; effect of the combination (zerumbone+caffeine+Globularia cordifolia extract) (n=4).**

| **Concentrations** | **MCP-1 (ng/10⁶ cell.)** | **MIF (ng/10⁶ cell.)** | **IL-6 (ng/10⁶ cell.)** |
|---|---|---|---|
| **6ppm of zerumbone + 300ppm of caffeine + *3%* of *Globularia cordifolia* extract)** | -34%; *p*<*0.01* | -40%; *p*<*0.01* | -77%; *p*<*0.01* |

The results show that the reduced attraction of macrophages by mature adipocytes (-66%, p<0.01; Table 24) appears to be explained by a co-ordinated reduction in molecules which attract macrophages and make them aggressive (MCP-1, MIF, IL-6; Table 26) obtained with the combination (zerumbone+caffeine+*Globularia cordifolia* extract).

Further more, by promoting adipocyte synthesis of adiponectin (+73%, p<0.01; table 25) and suppressing the synthesis of IL-6 in this cell, the combination also limits the macrophage attraction. This therefore results in reduced capacity of the macrophages to develop their inflammatory potential, induce the synthesis of proteases which degrade the matrix or promote adipocyte expansion, which leads to premature ageing of the adipose tissue.

### F) Galenic

Various galenic formulations are described below comprising zerumbone as slimming active molecule.

Furthermore, these formulations can also contain additional cosmetic active ingredients, the latter coming for each case in support and/or complement of the activity of the active ingredient according to the invention. These ingredients can be of any class according to their(s) function(s), site of application (body, face, neck, chest, hands, etc.), the desired end effect and the target consumer, in particular anti-aging, hydrating, firming, anti-redness, anti-stretch marks, etc.

Active ingredient of the invention: 500 ppm of zerumbone obtained by CO₂ supercritical extraction of *Zingiber zerumbet* (L.) Smith, prepared according to above example A in a butylen glycol matrix.

### Example 1: Slimming cream gel

| **Ingredient** | % | **INCI name** |
|---|---|---|
| **Phase A** | | |
| H₂O | Qsp100 | Water |
| Optasense G83 | 0.40 | Carbomer |

| **Phase B** | | |
|---|---|---|
| Crodacol CS 90 | 2.00 | Cetearyl Alcohol |
| Crodamol GTCC | 3.00 | Caprylic/Capric Triglyceride |

| **Phase C** | | |
|---|---|---|
| Butylene glycol | 4.00 | Butylene glycol |
| Phenoxyethanol | 1.00 | Phenoxyethanol |

| **Phase D** | | |
|---|---|---|
| Optasense G82 | 0.20 | Acrylic Acid/Alkylmethacrylate Copolymer |
| DC 200 5 cps | 3.00 | D imethic one |

| **Phase E** | | |
|---|---|---|
| Potassium sorbate | 0.10 | Potassium Sorbate |

| **Phase F** | | |
|---|---|---|
| H₂O | 6.00 | Water |
| NaOH 30% | 0.60 | Sodium Hydroxide |

| **Phase G** | | |
|---|---|---|
| Zerumbone extracted from *Zingiber zerumbet* (L.) Smith | 4.00 | / |

| **Phase H** | | |
|---|---|---|
| Tween 20 | 1.00 | Polysorbate 20 |
| Perfume | 0.10 | Fragrance |

**Protocol:** Phase A: Sprinkle carbomer in water and let swell without stirring 60 min. Heat phase A at 75°C in a water bath. Weigh phase B, mix and heat at 75°C in a water bath. Weigh phase C and mix. Weigh phase D and mix. Add phase C into phase A under propeller stirring. Add phase B and phase D into phase A+C fast propeller stirring. Add phase E, extemporaneously, in phase A+B+C+D, then mix well under blade stiring for 1 hour. Neutralize with phase F by pouring in the previous phase, mix well. Check the pH around 6. Add phase G at 35°C, mix well. Weigh and add phase H at 35°C, mix well.

### Example 2: Slimming massage cream

| **Ingredient** | **%** | **INCI name** |
|---|---|---|
| **Phase A** | | |
| H₂O | Qsp100 | Water |
| Ultrez 10 | 0.25 | Carbomer |

| **Phase B** | | |
|---|---|---|
| Hydrolite-5 | 5.00 | Pentylene Glycol |
| Phenoxyethanol | 1.00 | Phenoxyethanol |
| Keltrol CG-SFT | 0.25 | Xanthan Gum |

| **Phase C** | | |
|---|---|---|
| Brij S2 | 0.40 | Steareth-2 |
| Brij S10 | 1.20 | Steareth-10 |
| Crodafos CES | 4.00 | Cetearyl Alcohol & Dicetyl Phosphate & Ceteth-10 Phosphate |
| Crodamol CSO | 2.50 | Cetearyl Ethylhexanoate |
| BRB CM 56 | 2.00 | Cyclohexasiloxane & Cyclopentasiloxane |
| Crodamol OSU | 7.00 | Diethylhexyl Succinate |
| Crodacol CS 90 | 1.50 | Cetearyl Alcohol |

| **Phase D** | | |
|---|---|---|
| Potassium sorbate | 0.10 | Potassium Sorbate |

| **Phase E** | | |
|---|---|---|
| H₂O | 4.00 | Water |
| NaOH 30 % | 0.40 | Sodium Hydroxide |

| **Phase F** | | |
|---|---|---|
| Zerumbone extracted from *Zingiber zerumbet* (L.) Smith | 4.00 | / |

**Protocol:** Phase A: Sprinkle carbomer in water, let swell 30 minutes. Heat phase A at 75°C in a water bath. Weigh and mix phase B. Weigh phase C and heat at 75°C in a water bath. Add phase B into phase A under staro stirring. Pour phase C into phase A+B under staro stirring. Mix well. Add phase D, extemporaneously. Neutralize to pH = 6 with phase E below 35°C. Add phase F, mix well.

**Examples of additional ingredients that can be added to these formulations (marketed by Sederma):** REVIDRATE™: hydration active ingredient; BODYFIT™: slimming/firming active ingredient.

**These two creams, given by of illustration of the invention, may contain as variant** instead of the cosmetic ingredient based on an extract of *Zingiber zerumbet* (L.) Smith, a cosmetic ingredient according to the invention comprising the preferred association according to the invention of an extract of *Zingiber zerumbet* (L.) Smith and a *Globularia cordifolia* extract with, for example: in butylene glycol, 500ppm of zerumbone (obtained by CO₂ supercritical extraction of *Zingiber zerumbet* (L.) Smith according to above Example A) and 25% by weight of the extract of *Globularia cordifolia* (of an extract prepared according to above Example B at 33%).
0.75% of caffeine may also be added to this ingredient.

## Claims

1. Use of zerumbone for a non therapeutical cosmetic topical slimming treatment.

2. Use according to claim 1, to reduce expansion and/or prevent the development of adipose tissue; and/or to reduce the number and volume of adipocytes; and/or to activate the combustion of fatty acids and/or for a lipolytic treatment and/or for the treatment of cellulite, dimpling of skin and/or orange peel skin.

3. Use according to claim 1 or 2, for an anti-aging treatment of adipose tissue, in particular *via* the stimulation of elastin by the adipocytes.

4. Use according to one of claims 1 to 3, wherein zerumbone is combined with a *Globularia cordifolia* extract.

5. Use according to claim 4, wherein the *Globularia cordifolia* extract is obtained by *in vitro* plant cell culture.

6. Use according to one of the claims 1 to 5, wherein zerumbone is combined with a lipolytic agent.

7. Use according to claim 6, wherein the lipolytic agent is of the methylxanthine family selected from caffeine, theophylline, theobromine or aminophylline.

8. Use according to claim 7, wherein the lipolytic agent is caffeine.

9. Cosmetic topical composition comprising in a physiologically acceptable medium, zerumbone and at least one compound chosen among a *Globularia cordifolia* extract and a lipolytic agent.

10. Composition according to claim 9, **characterised in that** it comprises zerumbone, a *Globularia cordifolia* extract and a lipolytic agent.

11. Composition according to claim 9 or 10, wherein the *Globularia cordifolia* extract is obtained by *in vitro* plant cell culture.

12. Composition according to one of the claims 9 to 11, wherein the lipolytic agent is of the methylxanthine family selected from caffeine, theophylline, theobromine or aminophylline.

13. Composition according to claim 12, wherein the lipolytic agent is caffeine.

14. Composition according to claim 13, **characterised in that** it comprises zerumbone, a *Globularia cordifolia* extract and caffeine.

15. Use of a composition according to one of the claims 9 to 14 for a non therapeutical cosmetic topical treatment.

16. Woven or non-woven fabric material comprising zerumbone for a topical use according to anyone of claims 1 to 8 or according to claim 15.

17. Woven or non-woven material fabric comprising a composition according to anyone of claims 9 to 14.

## Patentansprüche

1. Verwendung von Zerumbon für eine nichttherapeutische kosmetische topische Schlankheitsbehandlung.

2. Verwendung nach Anspruch 1, um die Ausdehnung von Fettgewebe zu verringern und/oder die Entwicklung von Fettgewebe zu verhindern; und/oder um die Anzahl und das Volumen von Adipozyten zu verringern; und/oder um die Verbrennung von Fettsäuren zu aktivieren und/oder für eine lipolytische Behandlung und/oder zur Behandlung von Cellulitis, Hautvertiefungen und/oder Orangenhaut.

3. Verwendung nach Anspruch 1 oder 2 für eine Anti-Aging-Behandlung von Fettgewebe, insbesondere *durch* Stimulierung von Elastin durch die Adipozyten.

4. Verwendung nach einem der Ansprüche 1 bis 3, wobei Zerumbon mit einem *Globularia cordifolia-Extrakt* kombiniert wird.

5. Verwendung nach Anspruch 4, wobei der *Globularia cordifolia-Extrakt* durch eine *in vitro* Pflanzenzellkultur erhalten wird.

6. Verwendung nach einem der Ansprüche 1 bis 5, wobei Zerumbon mit einem lipolytischen Mittel kombiniert wird.

7. Verwendung nach Anspruch 6, wobei das lipolytische Mittel aus der Methylxanthin-Familie, ausgewählt aus Koffein, Theophyllin, Theobromin oder Aminophyllin, stammt.

8. Verwendung nach Anspruch 7, wobei das lipolytische Mittel Koffein ist.

9. Kosmetische topische Zusammensetzung, die ein physiologisch verträgliches Medium, Zerumbon und mindestens eine Verbindung, ausgewählt aus einem *Globularia cordifolia-Extrakt* und einem lipolytischen Mittel, umfasst.

10. Zusammensetzung nach Anspruch 9, **dadurch gekennzeichnet, dass** sie Zerumbon, einen *Globularia cordifolia-Extrakt* und ein lipolytisches Mittel umfasst.

11. Zusammensetzung nach Anspruch 9 oder 10, wobei der *Globularia cordifolia-*Extrakt durch eine *in vitro* Pflanzenzellkultur erhalten wird.

12. Zusammensetzung nach einem der Ansprüche 9 bis 11, wobei das lipolytische Mittel aus der Methylxanthin-Familie, ausgewählt aus Koffein, Theophyllin, Theobromin oder Aminophyllin, stammt.

13. Zusammensetzung nach Anspruch 12, wobei das lipolytische Mittel Koffein ist.

14. Zusammensetzung nach Anspruch 13, **dadurch gekennzeichnet, dass** sie Zerumbon, einen *Globularia cordifolia*-Extrakt und Koffein enthält.

15. Verwendung einer Zusammensetzung nach einem der Ansprüche 9 bis 14 für eine nichttherapeutische kosmetische topische Behandlung.

16. Gewebe oder Vliesmaterial, umfassend Zerumbon zur topischen Verwendung nach einem der Ansprüche 1 bis 8 oder nach Anspruch 15.

17. Gewebe oder Vliesmaterial, umfassend eine Zusammensetzung nach einem der Ansprüche 9 bis 14.

## Revendications

1. Utilisation de la zerumbone pour un traitement topique non-thérapeutique cosmétique amincissant.

2. Utilisation selon la revendication 1, pour diminuer l'expansion et/ou en prévenir le développement de tissu adipeux ; et/ou pour réduire le nombre et le volume des adipocytes ; et/ou pour activer la combustion des acides gras et/ou pour un traitement lipolytique et/ou pour le traitement de la cellulite, des capitons et/ou de la peau d'orange.

3. Utilisation selon la revendication 1 ou 2, pour un traitement anti-âge du tissu adipeux, en particulier *via* la stimulation d'élastine par les adipocytes.

4. Utilisation selon l'une des revendications 1 à 3, dans laquelle la zerumbone est combinée à un extrait de *Globularia Cordifolia.*

5. Utilisation selon la revendication 4, dans laquelle l'extrait de *Globularia cordifolia* est obtenu par culture végétale cellulaire *in vitro.*

6. Utilisation selon l'une des revendications 1 à 5, dans laquelle la zerumbone est combinée à un agent lipolytique.

7. Utilisation selon la revendication 6, **caractérisée en ce que** l'agent lipolytique est de la famille des méthylxanthines choisi parmi la caféine, la théophylline, la théobromine ou l'aminophylline.

8. Utilisation selon la revendication 7, dans laquelle l'agent lipolytique est la caféine.

9. Composition cosmétique topique comprenant dans un milieu physiologiquement acceptable, la zerumbone et au moins un composé choisi parmi un extrait de *Globularia Cordifolia* et un agent lipolytique.

10. Composition selon la revendication 9, **caractérisée en ce qu'**elle comprend la zerumbone, un extrait de *Globularia Cordifolia* et un agent lipolytique.

11. Composition selon la revendication 9 ou 10, dans laquelle l'extrait de *Globularia cordifolia* est obtenu par culture végétale cellulaire *in vitro.*

12. Composition selon l'une des revendications 9 à 11, dans laquelle l'agent lipolytique est de la famille des méthylxanthines choisi parmi la caféine, la théophylline, la théobromine ou l'aminophylline.

13. Composition selon la revendication 12, dans laquelle l'agent lipolytique est la caféine.

14. Composition selon la revendication 13, **caractérisée en ce qu'**elle comprend la zerumbone, un extrait de *Globularia Cordifolia* et la caféine.

15. Utilisation d'une composition selon l'une des revendications 9 à 14 pour un traitement topique non-thérapeutique cosmétique.

16. Matériau tissé ou non-tissé comprenant de la zerumbone, pour une utilisation topique selon l'une quelconque des revendications 1 à 8 ou selon la revendication 15.

17. Matériau tissé ou non-tissé comprenant une composition selon l'une des revendications 9 à 14.


### SEQUENCE LISTING

<110> SEDERMA FOURNIAL, ARNAUD GRIZAUD, CLAIRE-MARIE MONDON, PHILIPPE
<120> NEW USE OF ZERUMBONE AND COMPOSITIONS COMPRISING ZERUMBONE
<130> ZERCO PCT
<150> FR1250241
   <151> 2012-01-10
<150> FR1256579
   <151> 2012-07-09
<160> 10
<170> PatentIn version 3.5
<210> 1
   <211> 6
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 1
   Val Gly Val Ala Pro Gly
   1               5
<210> 2
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 2
   Lys Thr Thr Lys Ser
   1               5
<210> 3
   <211> 4
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 3
   Gly Gln Pro Arg
   1
<210> 4
   <211> 4
   <212> PRT
   <213> artificial sequence
<220>
   <223> synthetic peptide
<400> 4
   Arg Ser Arg Lys
   1
<210> 5
   <211> 4
   <212> PRT
   <213> artificial sequence
<220>
   <223> synthetic peptide
<400> 5
   Gly Gln Pro Arg
   1
<210> 6
   <211> 4
   <212> PRT
   <213> artificial sequence
<220>
   <223> synthetic peptide
<400> 6
   Lys Thr Phe Lys
   1
<210> 7
   <211> 5
   <212> PRT
   <213> artificial sequence
<220>
   <223> synthetic peptide
<400> 7
   Lys Thr Thr Lys Ser
   1 5
<210> 8
   <211> 6
   <212> PRT
   <213> artificial sequence
<220>
   <223> Synthetic peptide
<400> 8
   Gly Lys Thr Thr Lys Ser
   1 5
<210> 9
   <211> 6
   <212> PRT
   <213> artificial sequence
<220>
   <223> synthetic peptide
<400> 9
   Val Gly Val Ala Pro Gly
   1 5
<210> 10
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<220>
   <221> MISC_FEATURE
   <222> (5)..(5)
   <223> Xaa being either a Methionine M or a Leucine L.
<400> 10 SEQUENCE LISTING
<110> SEDERMA FOURNIAL, ARNAUD GRIZAUD, CLAIRE-MARIE MONDON, PHILIPPE
<120> NEW USE OF ZERUMBONE AND COMPOSITIONS COMPRISING ZERUMBONE
<130> ZERCO PCT
<150> FR1250241
   <151> 2012-01-10
<150> FR1256579
   <151> 2012-07-09
<160> 10
<170> PatentIn version 3.5
<210> 1
   <211> 6
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 1
   Val Gly Val Ala Pro Gly
   1 5
<210> 2
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 2
   Lys Thr Thr Lys Ser
   1 5
<210> 3
   <211> 4
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 3
   Gly Gln Pro Arg
   1
<210> 4
   <211> 4
   <212> PRT
   <213> artificial sequence
<220>
   <223> synthetic peptide
<400> 4
   Arg Ser Arg Lys
   1
<210> 5
   <211> 4
   <212> PRT
   <213> artificial sequence
<220>
   <223> synthetic peptide
<400> 5
   Gly Gln Pro Arg
   1
<210> 6
   <211> 4
   <212> PRT
   <213> artificial sequence
<220>
   <223> synthetic peptide
<400> 6
   Lys Thr Phe Lys
   1
<210> 7
   <211> 5
   <212> PRT
   <213> artificial sequence
<220>
   <223> synthetic peptide
<400> 7
   Lys Thr Thr Lys Ser
   1               5
<210> 8
   <211> 6
   <212> PRT
   <213> artificial sequence
<220>
   <223> Synthetic peptide
<400> 8
   Gly Lys Thr Thr Lys Ser
   1               5
<210> 9
   <211> 6
   <212> PRT
   <213> artificial sequence
<220>
   <223> synthetic peptide
<400> 9
   Val Gly Val Ala Pro Gly
   1               5
<210> 10
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<220>
   <221> MISC_FEATURE
   <222> (5)..(5)
   <223> Xaa being either a Methionine M or a Leucine L.
<400> 10
   Tyr Gly Gly Phe Xaa
   1               5